Europäisches Patentamt

European Patent Office

Office européen des brevets

(11) Publication number: **0 084 344**
**A2**

# EUROPEAN PATENT APPLICATION

(21) Application number: 83100190.4

(22) Date of filing: 12.01.83

(51) Int. Cl.³: **C 12 N 15/00**
C 12 P 1/00, G 01 N 33/54
C 12 N 9/72
//C12R1/91, C12N5/00

(30) Priority: 14.01.82 JP 3290/82
19.08.82 JP 142677/82
26.08.82 JP 146822/82

(43) Date of publication of application:
27.07.83 Bulletin 83/30

(84) Designated Contracting States:
DE FR GB IT SE

(71) Applicant: Asahi Kasei Kogyo Kabushiki Kaisha
2-6, Dojimahama 1-chome Kita-ku
Osaka-shi Osaka 530(JP)

(72) Inventor: Hasegawa, Akio
5-24, Sugizakicho Numazu-shi
Shizuoka-ken(JP)

(72) Inventor: Yamashita, Hitoshi
Asahi-Kasei-Dai-5-Ryo 100, Kawanarijima
Fuji-shi Shizuoka-ken(JP)

(74) Representative: Werner, Hans-Karsten, Dr. et al,
Deichmannhaus am Hauptbahnhof
D-5000 Köln 1(DE)

(54) Anti-urokinase monoclonal antibodies and process for preparing the same.

(57) An anti-urokinase monoclonal antibody which belongs to IgGI subclass and having a specificity for human urokinase, a molecular weight of 140,000-180,000 and an isoelectric point of 5.0-6.5.

FIG.1

EP 0 084 344 A2

## BACKGROUND OF THE INVENTION

### Field of the Invention

This invention relates to the production of monoclonal antibodies specific for urokinase, and in particular, human urokinase having a molecular weight of 54,000 daltons. The invention provides a method for repeatedly producing large quantities of identical antibodies against urokinase which may be used for diagnostic, analytical and/or purification purposes.

### Description of the Prior Art

Antibodies having specificity for a particular antigen have been obtained from the sera of animals immunized with the antigen. However, these antibodies from conventional antisera have only restricted utility because they constitute a collection of many different antibodies, each specific for the antigen, i.e., they are polyclonal. This collection of different antibodies is produced in response to immunization with a single antigen because antigens contain a multiplicity of different antigenic sites (antigenic determinants), each of which can give rise to a single type of antibody. Additionally, it is very difficult to separate from this polyclonal mixture a uniform collection of antibodies against a single antigenic determinant.

Urokinase is a fibrinolytic enzyme found in human urine and the culture fluid of cells derived from a human. There are at least two active forms of the enzyme, the heavier form (54,000 daltons) and its derived lighter form (33,000 daltons). Medical preparations of both forms have been widely used for the treatment of thrombosis and myocardial infarction. Further, it has been shown that urokinase administered together with an anticancer agent is useful in the treatment of cancer. Accordingly, there is a strong desire in clinical medicine to obtain urokinase having high purity and free from substances which lead to undesirable side effects.

Urokinase has been purified by the methods using adsorbents such as barium sulfate, silicic acid and salts thereof, and activated carbon; various kinds of ion exchangers; insoluble carriers having lysine or arginine attached thereto [Japanese Patent Application (OPI) 125584/1974]; agarose, polyacrylamide and polysaccharide, each having cetraxate thereon [Japanese Patent Publication 40040/1980]; and insoluble carriers having heparin or its alkali metal salt on their surfaces [Japanese Patent Publication 39192/1981]. However, urokinase having very high purity has not been obtained in the above-described methods because the adsorbents, ion exchangers and carriers employed in the methods have relatively low affinity for urokinase. These methods are also not satisfactory from the view point of their low percentage yield of recovery of urokinase.

A method for purifying urokinase with the anti-urokinase antibodies obtained from conventional antiserum has been proposed, but the method has several disadvantages. First, since the antibody obtained from antiserum is a mixture of various antibodies (polyclonal), affinity of each antibody against urokinase is different. Second, the production of the antibody itself is not always reproducible. Third, the conditions for attaching the antibody to the carrier are not constant and, accordingly, the process for fixing the antibody to the carrier and the process for purifying urokinase are complicated. Further, the yield of recovery and the purity are not sufficiently high.

A method for preparing a hybridoma which can produce a monoclonal antibody was established by Milstein et al. as described in "Nature" Vol. 256, 495-497, 1975. Further, the methods for preparing the hybridomas have been reported by G. Kohler et al., "Somatic Cell Genetics" Vol. 3, 303, 1977; R.A. Goldsberg et al., "Nature" Vol. 267, 707, 1977; and Masaru Taniguchi et al., "Clinical Immunity" Vol. 12(4), 284-289, 1980.

Monoclonal antibodies against the 33,000 dalton species have been reported. P. Herion et al., "Bioscience Reports" Vol. 1, 885-892 (1981).

## SUMMARY OF THE INVENTION

The object of this invention is to provide novel anti-urokinase monoclonal antibodies, and methods for producing and using these monoclonal antibodies.

More particularly, this invention relates to novel anti-urokinase monoclonal antibodies which belong to IgG1 subclass and having (1) a specificity for urokinase derived from a human (54,000 dalton molecular weight species), (2) a molecular weight of about 140,000-180,000 daltons and (3) an isoelectric point of 5.0-6.5.

## BRIEF DESCRIPTION OF THE DRAWING

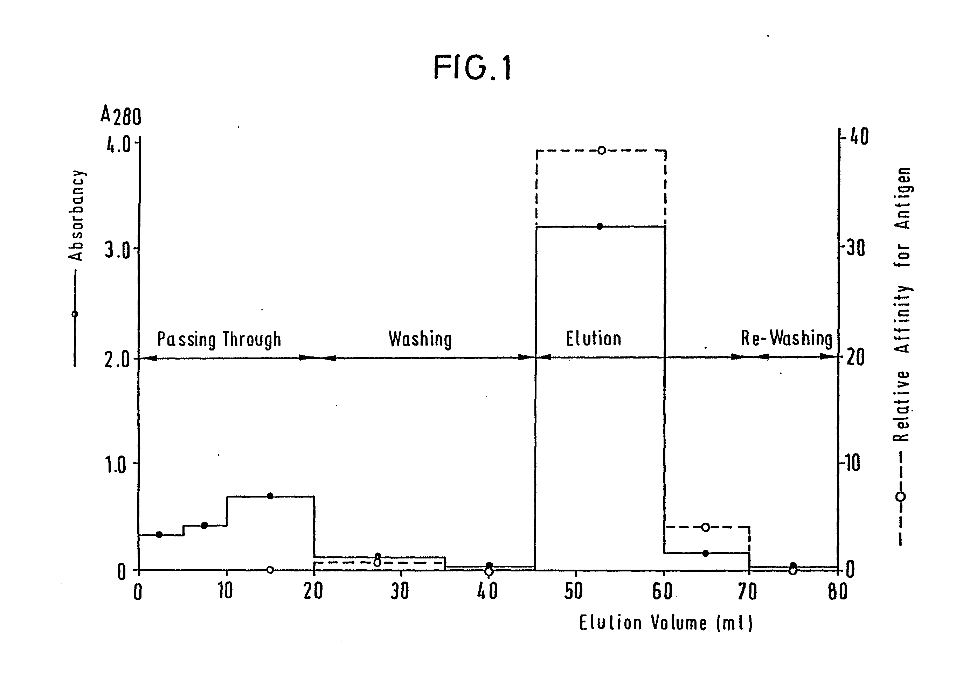

FIGS. 1 to 4 represent patterns of affinity chromatography obtained by carrying out the purification of anti-urokinase monoclonal antibodies produced in Examples 2 to 5, and show the relationship between the absorbancy at 280 nm or the relative affinity of the antibodies for urokinase and the elution volume.

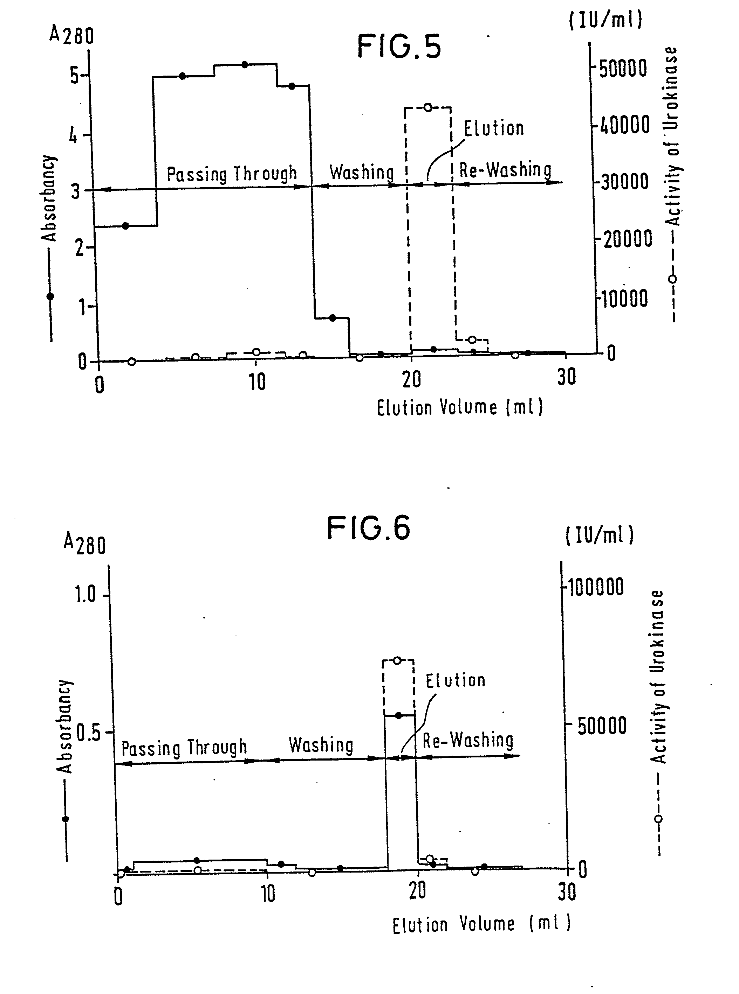

FIGS. 5 and 6 represent patterns of affinity chromatography obtained by carrying out the purification of urokinase with anti-urokinase monoclonal antibodies produced in Examples 6 and 10, and show the relationship between the absorbancy at 280 nm or the activity of urokinase and the elution volume.

## DETAILED DESCRIPTION OF THE INVENTION

The anti-urokinase monoclonal antibodies of this invention can be obtained by cultivating novel hybridomas which can produce the anti-urokinase monoclonal antibodies and recovering the antibodies. The hybridomas can be propagated in vivo in a histocompatible animal and the body fluids of the animal, such as serum or ascites fluid, may be harvested to provide antibodies in high concentration. Alternatively, the hybridomas may be propagated in vitro in laboratory vessels and the culture medium may be harvested to obtain the monoclonal antibodies.

The novel hybridomas producing the anti-urokinase monoclonal antibodies can be prepared by fusing mouse lymphocyte B cells with mouse myeloma cells in accordance with the following method with good reproducibility. In more detail, the hybridomas can be prepared by immunizing male mice BALB/c with urokinase (molecular weight 54,000 daltons); obtaining B cells from the spleens of the immunized mice; fusing the B cells with mouse myeloma cells, such as P3x63Ag8U1, obtained from the same kind of mouse in a ratio of 1 to 10 B cells per mouse myeloma cell in the presence of polyethylene glycol; suspending the fused cells in HAT medium [RPMI-1640 culture medium (Flow Laboratories, Inc., U.S.A) supplemented with 12mM hypoxanthine, 9µM aminopterin and 8mM thymidine] supplemented with 10% fetal calf serum; and plating the cells in 96-well dish. After about one week from the plating, most cells except the (fused) hybridomas of myeloma cells and B cells perish, and colonies of the hybridomas are produced.

The hybridomas are then examined to determine whether they produce antibodies against urokinase by assaying the supernatants of the culture media thereof in accordance with the method described in "Enzyme Immunoassay" (edited by Eiji Ishikawa et al., published by Igaku Shoin, 1978). That is, the supernatant from the culture medium is added to wells of 96-well dishes to which urokinase has been physically adsorbed to bind any anti-urokinase antibody contained in the supernatant. Then, after washing away any unbound material, peroxidase-conjugated anti-mouse IgG antibody, which can recognize the monoclonal antibody bound to urokinase, is added to bind the monoclonal antibody-urokinase complex. Then, after again washing the plate to remove unbound peroxidase-conjugated antibody, a substrate for peroxidase is added to the wells to determine whether the substrate can react with the peroxidase-conjugated antibody. If the reaction occurs, it confirms that the hybridomas are producing the antibodies to urokinase.

The colonies of the hybridomas producing antibodies to urokinase are then selected and cloned so as to cultivate one colony in one well. After 7-10 days, the colonies are examined again by the same enzyme immunoassay described above.

In the method described above, four kinds of hybridomas which produce the following four anti-urokinase monoclonal antibodies were obtained: 1G11a, 2C3i, 5G4i and 6G8u.

### 1G11a Anti-urokinase Monoclonal Antibody
Molecular Weight: 150,000-170,000
Isoelectric Point: 5.25-5.6
Amino Acid Composition:

| Amino Acid | Content (mol %) |
|---|---|
| Asparagine and Aspartic Acid | 10.0 |
| Threonine | 9.7 |
| Serine | 12.0 |
| Glutamine and Glutamic Acid | 11.0 |
| Glycine | 11.4 |
| Alanine | 6.2 |
| Valine | 7.0 |
| Cysteine* | Not Found |
| Methionine | Not Found |
| Isoleucine | 3.2 |
| Leucine | 5.7 |
| Tyrosine | 2.9 |
| Phenylalanine | 3.5 |
| Lysine | 6.3 |
| Histidine | 2.0 |
| Arginine | 2.3 |
| Proline | 6.9 |

---

* Cysteine changes into cystine when the cysteine is hydrolyzed and air-oxidized.

2C3i Anti-urokinase Monoclonal Antibody
Molecular Weight:   150,000-165,000
Isoelectric Point:   5.95-6.25
Amino Acid Composition:

| Amino Acid | Content (mol %) |
|---|---|
| Asparagine and Aspartic Acid | 8.0 |
| Threonine | 8.1 |
| Serine | 9.8 |
| Glutamine and Glutamic Acid | 8.2 |
| Glycine | 24.3 |
| Alanine | 5.0 |
| Valine | 6.4 |
| Cystine | Not Found |
| Methionine | 0.7 |
| Isoleucine | 3.3 |
| Leucine | 5.0 |
| Tyrosine | 2.6 |
| Phenylalanine | 3.0 |
| Lysine | 5.3 |
| Histidine | 1.8 |
| Arginine | 2.5 |
| Proline | 6.1 |

0084344

## 5G4i Anti-urokinase Monoclonal Antibody
### Molecular Weight: 150,000-170,000
### Isoelectric Point: 5.75-6.15
### Amino Acid Composition:

| Amino Acid | Content (mol%) |
|---|---|
| Asparagine and Aspartic Acid | 10.2 |
| Threonine | 9.4 |
| Serine | 11.4 |
| Glutamine and Glutamic Acid | 10.3 |
| Glycine | 8.6 |
| Alanine | 5.9 |
| Valine | 7.1 |
| Cystine | 1.1 |
| Methionine | 1.1 |
| Isoleucine | 3.3 |
| Leucine | 5.7 |
| Tyrosine | 3.4 |
| Phenylalanine | 3.9 |
| Lysine | 6.4 |
| Histidine | 2.1 |
| Arginine | 2.8 |
| Proline | 7.3 |

### 6G8u Anti-urokinase Monoclonal Antibody
Molecular Weight: 150,000-175,000
Isoelectric Point: 5.1-5.4
Amino Acid Composition:

| Amino Acid | Content (mol %) |
| --- | --- |
| Asparagine and Aspartic Acid | 7.6 |
| Threonine | 6.8 |
| Serine | 8.6 |
| Glutamine and Glutamic Acid | 8.4 |
| Glycine | 31.7 |
| Alanine | 4.7 |
| Valine | 2.3 |
| Cystine | 1.0 |
| Methionine | 1.0 |
| Isoleucine | 3.0 |
| Leucine | 4.9 |
| Tyrosine | 3.4 |
| Phenylalanine | 2.7 |
| Lysine | 5.0 |
| Histidine | 1.6 |
| Arginine | 2.0 |
| Proline | 5.4 |

Affinity for Diethylaminoethyl Ion Exchanger:

Binding in the presence of a solution having the conductivity of 0-15 mʊ/cm.

The hybridomas thus obtained continue producing the anti-urokinase monoclonal antibodies upon subculturing the hybridomas. The anti-urokinase monoclonal antibodies can be obtained by cultivating the hybridomas in RPMI-1640 culture medium containing 10 - 20% fetal calf serum and recovering the antibodies from the culture medium. Alternatively, the hybridomas may be propagated intraperitoneally by injecting from about $5 \times 10^6$ to about $1 \times 10^7$ cells of the hybridoma into BALB/c mouse primed with 0.2 mℓ of 2,6,10,14-tetramethylpentadecane (pristane), growing the hybridoma to give an ascites and recovering the antibodies from the ascites. The recovery of the anti-urokinase monoclonal antibodies from the culture media or the ascites can be carried out in accordance with the known method of recovering an antibody from serum, for example, described in L. Hudson et al., "Practical Immunology", Blackwell Sci. Pub., 1976. A preferred method for recovering the anti-urokinase monoclonal antibodies is affinity chromatography using a carrier chemically bonded with urokinase. More specifically, the solution containing the anti-urokinase monoclonal antibodies, such as culture media or ascites, is contacted with the carrier packed in a column to selectively remove the anti-urokinase monoclonal antibodies. Subsequently, the carrier is contacted with an eluent to elute the anti-urokinase monoclonal antibody from the carrier. The separation of the anti-urokinase monoclonal antibody from the eluent can be carried out by a conventional method such as dialysis.

The carriers which can be employed in the recovery of the anti-urokinase monoclonal antibodies include polysaccharides, such as Sepharose (Pharmacia Fine Chemicals Co.), agarose, cellulose and dextran; polyacrylamide; and porous glass, which are used in conventional affinity chromatography.

Urokinase can be chemically bound to the carrier by activating the carrier with an agent such as cyanogen bromide, epoxide or periodic acid, or by activating amino groups of a carrier with glutaraldehyde prior to formation of a covalent bond.

The eluents which can be employed in the recovery of the anti-urokinase monoclonal antibodies include phosphoric acid-citric acid buffer solution (pH 2.8), propionic acid, aqueous ammonia solution (pH 11), glycine-hydrochloric acid buffer solution (pH 2.8), chaotropic ion, aqueous sodium thiocyanate solution, guanidine-hydrochloric acid buffer solution (pH 3.1), 8M aqueous urea solution (pH 7.0) and ethylene glycol (pH 11.5), which are used in conventional affinity chromatography.

The recovery methods using the carriers which are bonded with urokinase have the following advantages and are industrially useful:

(1)   Antibodies against antigens other than urokinase contained in anti-urokinase monoclonal antibody-containing solution can be easily removed.

(2)   Anti-urokinase monoclonal antibodies having high purity can be obtained.

(3)   Since the anti-urokinase monoclonal antibodies can be adsorbed to the carrier in a very short time as compared with the methods such as ion-exchange chromatography and gel filtration, the operation time for the recovery can be significantly shortened.

(4)   No operational difficulties are encountered because the size of the column can be reduced due to the large adsorption capacity of the carrier for anti-urokinase monoclonal antibodies.

(5)   The carrier to which the urokinase is bound can be repeatedly used by washing the carrier with a washing solution after the antibodies are desorbed, and therefore, the recovery operation is simple.

The anti-urokinase monoclonal antibodies of this invention have specificity for urokinase and are useful for purifying urokinase and for various diagnostic and analytical utilities.

The purification of urokinase can be carried out by binding the anti-urokinase monoclonal antibodies of this invention to a carrier; contacting a fluid containing urokinase, such as urine, a culture fluid of cells derived from human being, a culture fluid of a microorganism capable of producing urokinase, or partially purified solutions thereof, with the carrier (usually packed in a column); washing the carrier with a washing liquid of pH 6-8, such as a physiological salt solution, to remove unadosorbed impurities; and eluting urokinase from the carrier with an eluent.

Any carriers, methods for binding the antibodies with carriers and eluents which have been employed in conventional affinity chromatography can be used in the purification of urokinase described above.

The purification methods using the carriers which are bonded with anti-urokinase monoclonal antibodies of this invention can easily remove impurities contained in a urokinase-containing solution in a single step. Further, urokinase having a high purity of at least 100,000 IU/mg can be obtained with a high recovery yield of at least 90% in this invention. When at least two anti-urokinase monoclonal antibodies of this invention are employed together in the purification, urokinase having even higher purity can be obtained.

The anti-urokinase monoclonal antibodies of this invention can be clinically used for immunological determination of urokinase in a fluid, such as blood. On the other hand, many unresolved scientific questions still remain with regard to urokinase, such as the working mechanism in blood, the relation between the dosage and the effect, the relationship with other plasminogen activators and so on. The anti-urokinase monoclonal antibodies of this invention may be utilized in the study of blood coagulation and fibrinolysis are ideally suited for such use because the antibodies of this invention can detect urokinase more sensitively and more selectively than conventional mixed antibodies. Therefore, the blood level of urokinase can be accurately measured even if only a small amount of the antibodies of this invention is employed.

The physicochemical properties of the antibodies, shown in this invention were measured in the following methods.

Molecular Weight:

An SDS-polyacrylamide gel electrophoresis was employed in accordance with the method described in U.K. Laemmli, "Nature" Vol. 227, 680, 1970, using 9% gel. The antibodies were measured in the non-reduced state.

IgG Subclass:

On 96-well microtiter plate were placed rabbit anti-mouse IgG1, IgG2a and IgG2b (Miles Co.) which were diluted 1,000 times with physiological salt solution. To each of the rabbit anti-mouse IgG1, IgG2a and IgG2b wells was added an antibody, and the mixtures were left to stand one night at 4°C. Then, the IgG subclass was determined in accordance with the enzyme immunoassay described above.

## Isoelectric Point:

A thin layer gel isoelectrofocusing method was employed in the measurement. During the measurent, Pharmalyte (Pharmacia Fine Chemicals Co.) having a pH of 3-10 was used as an ampholyte, and the measurement was carried out in accordance with the method described in Z.L. Awdeh et. al., "Nature", Vol. 219, 66-67, 1968.

## Amino Acid Composition:

Antibody was hydrolyzed with 6N HCl at 110°C under vacuum for 24 hours, and the amino acid composition thereof was measured by a High-Speed Amino Acid Analyzer (Hitachi Co.; Type 835).

## Affinity for Diethylaminoethyl (DEAE) Ion Exchanger:

In 0.025M tris-hydrochloric acid buffer solutions (pH 8.0) having conductivities of 0.5, 10 and 15 m℧/cm, antibody was contacted with diethylaminoethyl cellulose [trade name DE 52; Whatman Co.] with stirring. The amount of antibody remaining in the supernatant was measured as absorbancy at 280 nm, and the amount of bound antibody was calculated by difference from the absorbancy. The binding to DEAE ion exchanger was represented as a ratio of the amount of bound antibody to the total amount of the antibody.

The following examples illustrate the present invention in more detail, but they are given for illustrative purposes only and are not to be construed as limiting the invention.

## Example 1

A culture medium of human fetal kidney cells was purified to obtain urokinase solution (54,000 dalton molecular weight species) having a specific activity of 56,700 IU/mg and a protein concentration of 2 mg/ml. The urokinase solution was diluted with an equal amount of a complete Freund's adjuvant and well mixed.

A male BALB/c mouse, age 6 weeks, was subcutaneously injected with 0.2 ml of the diluted urokinase solution every two weeks for immunization. Four days after the fourth injection, the spleen was removed, passed through a stainless steel mesh to obtain dispersed cells, and then suspended in a Dulbecco's modified minimal essential medium (D-MEM) medium. Both spleen cells thus obtained and mouse myeloma cells (P3x63Ag8UI) originating from the same kind of mouse were washed twice with the D-MEM medium and were centrifuged at 1000xg for 5 minutes. Each precipitate was suspended again in the D-MEM medium. After the number of cells was counted in a blood cell counting chamber, $2 \times 10^8$ spleen cells were mixed (in a 50 ml conical tube) with $2 \times 10^7$ myeloma cells. After mixing well, the mixture was centrifuged and the medium was removed with suction. The conical tube was tapped to loosen the pellet of cells, and heated in a water bath maintained at 37°C. To the cells were then added dropwise 1 ml of a 45% by weight polyethylene glycol solution ("PEG-4000", Sigma Co.) heated at 37°C while shaking the conical tube. After the tube was left to stand 5 minutes, 40 ml of the D-MEM medium were added to the mixture of the cells and the polyethylene glycol solution to dilute the polyethylene glycol. The mixture was centrifuged and the supernatant was removed with suction. Then, to the mixture was added slowly 50 ml of HAT medium heated to 37°C and containing 10% fetal calf serum to disperse the cells. About 0.1 ml of the dispersion was poured into each well of 96-well culture plates. One week later, colonies of hybridomas were present in the wells.

Urokinase having a concentration of 20 µg/ml was placed and adsorbed onto the surface of wells of 96-well microtiter plates for enzyme immunoassay (EIA microtiter plates) at 4°C for one night. Then, excess bovine serum albumin was added to the wells which were then washed with a physiological salt solution. On each well of the EIA microtiter plates was placed the supernatant of the clone of a hybridoma and the reactions were carried out at 25°C for one hour. After the reactions were completed, the wells were fully washed with a physiological salt solution. To each well was added 50 µl of a peroxidase-conjugated anti-mouse immunoglobulin solution as the secondary antibody and the reactions were carried out at 25°C for one hour. After the solution was removed with a suction, the wells were washed with a physiological salt solution. To each well were added 150 µl of a 0.3% by weight o-phenylenediamine and 150 µl of a 0.1M citrate buffer solution (pH 5.0) containing 0.03% by weight $H_2O_2$. Then, the reactions were carried out at 37°C for 15 minutes in the dark. The absorbancy of the solutions in the wells were measured at 492 nm by Titertek Multi Skan (spectrophotometer; product of Dainippon Pharmaceutical Co., Ltd.). As the result of the measurement, the enzyme reaction was observed for 243 colonies in the 98 wells. Each of the colonies was sucked with a micropipet while watching the colony under a microscope and transferred to a well of 96-well culture plates. To each colony in the well was added a HAT medium containing 10% fetal calf serum and the cultivation was carried out for 8 days. Production of antibody by the hybridomas thus cloned was examined again by the same enzyme immunoassay as described above. As a result, 74 hybridomas which could produce anti-urokinase antibodies were obtained. These 74 hybridomas were subcultured on 6-well culture plates and further subcultured on 100 mm dishes. As the result of the subculture, 35 hybridomas which could produce anti-urokinase antibodies in the supernatant of the culture and had good multiplication were obtained. It was found that out of 35 hybridomas, at least 2 hybridomas produced lGlla anti-urokinase monoclonal antibody, at least 2 hybridomas produced 2C3i anti-urokinase monoclonal antibody, at least one hybridoma produced 5G4i anti-urokinase monoclonal antibody and at least 2 hybridomas produced 6G8u anti-urokinase monoclonal antibody.

## Example 2

The hybridoma which can produce the lGlla anti-urokinase monoclonal antibody of this invention was intraperitoneally cultured in BALB/c mouse to give ascites. To 4 mℓ of the ascites obtained was added ammonium sulfate to make 45% saturation, and the mixture was centrifuged to give a precipitate. The precipitate was dissolved in 4 mℓ of a physiological phosphate buffer solution (pH 7.0), and the solution was dialyzed in 800 mℓ of the same buffer solution at 4°C for one night. After the dialysis, the solution was centrifuged to give a crudely purified antibody solution as the precipitate. The antibody solution was passed through a 15 mℓ column packed with Sepharose (Pharmacia Fine Chemicals Co.)

having been chemically bonded with 2.4 mg of urokinase per milliliter of Sepharose after the column was throughly washed with a physiological phosphate buffer solution.

After the unadsorbed fraction was removed by washing the column with a physiological phosphate buffer solution, 1G11a anti-urokinase monoclonal antibody was eluted from the column with a 0.1M glycine-hydrochloric acid buffer solution (pH 2.8). The absorbancy of the eluted fraction was measured at 280 nm and the relative affinity of the eluted fraction for urokinase was measured in accordance with the enzyme immunoassay described above. The results are shown in FIG. 1.

Almost all of the active antibody was found in the eluted fraction as shown in FIG. 1. Further, it was confirmed that the eluted fraction exhibited a single band in a polyacrylamide gel electrophoresis.

## Example 3

Two liters of the culture fluid obtained by cultivating the hybridoma which can produce the 2C3i anti-urokinase monoclonal antibody of this invention was treated with ammonium sulfate and dialyzed in the same manner as in Example 2 to give 50 milliliter of crudely purified antibody solution. The antibody solution was passed through a 12 mℓ column packed with the grannular polyacrylamide having been chemically bonded with 2 mg of urokinase per milliliter of the polyacrylamide after the column was thoroughly washed with a physiological phosphate buffer solution (pH 7.0).

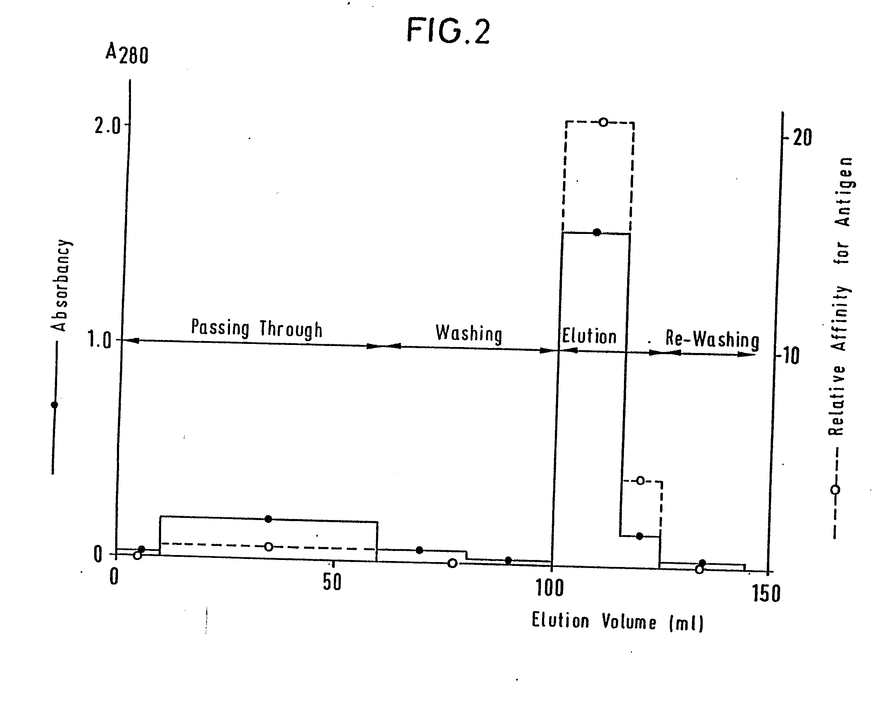

After the unadsorbed fraction was removed by washing the column with a physiological phosphate buffer solution, 2C3i anti-urokinase monoclonal antibody was eluted from the column with a 4% by weight $NH_4OH$ solution (pH 11). The absorbancy of the eluted fraction at 280 nm and the relative affinity of the eluted fraction for urokinase were measured, and are shown in FIG. 2.

As shown in FIG. 2, almost all of the active antibody was found in the eluted fraction. Further, it was confirmed that the eluted fraction exhibited a single band in an electrophoresis.

## Example 4

The hybridoma which can produce the 5G4i anti-urokinase monoclonal antibody of this invention was intraperitoneally cultured in BALB/c mouse to give ascites. 4.5 mℓ of the ascites obtained was passed through a 10 mℓ column packed with granular dextran having been chemically bonded with 3 mg of urokinase per milliliter of dextran after the column was thoroughly washed with 10mM of a phosphate buffer solution (pH 7.0).

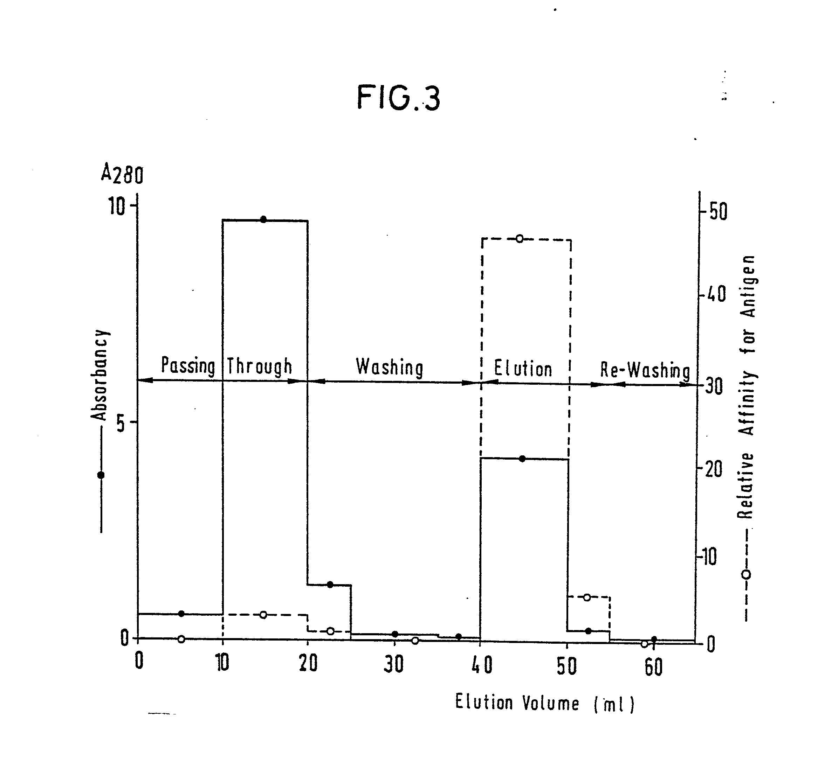

After the unadsorbed fraction was removed by washing the column with 10mM of a phosphate buffer solution, 5G4i anti-urokinase monoclonal antibody was eluted from the column with an 8M aqueous urea solution (pH 7.0). The absorbancy of the eluted fraction at 280 nm and the relative affinity of the eluted fraction for urokinase were measured, and their relation is shown in FIG. 3.

As shown in FIG. 3, almost all of the active antibody was found in the eluted fraction. Further, it was confirmed that the eluted fraction exhibited a nearly single band in an electrophoresis.

## Example 5

The hybridoma which can produce the 6G8u anti-urokinase monoclonal antibody of this invention was intraperitoneally cultured in BALB/c mouse. Five mℓ of the ascites thus obtained was purified in the same manner as in Example 2 by using the column packed with Sepharose having been bonded with urokinase.

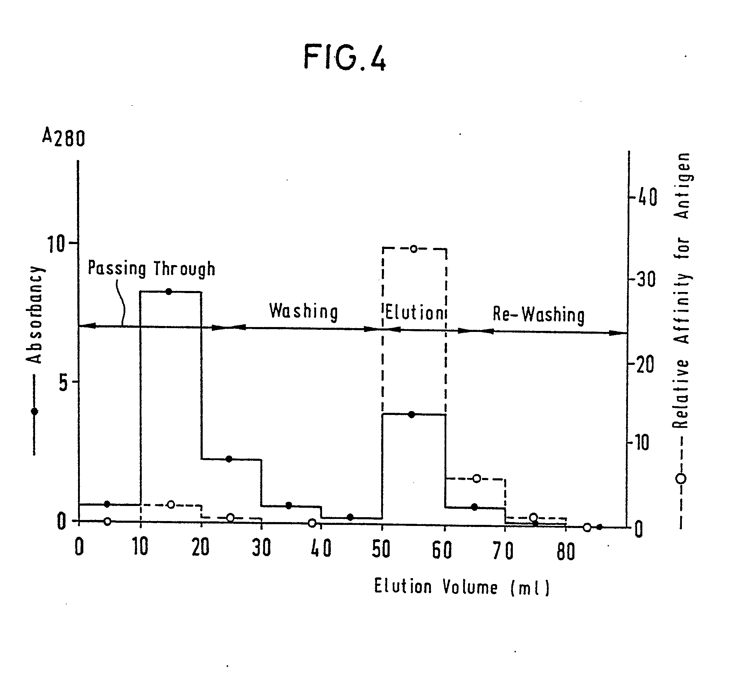

The pattern of chromatography obtained is shown in FIG. 4. Almost all of the active antibody was found in the eluted fraction. Further, it was confirmed that the recovery specimen exhibited a nearly single band in an electrophoresis.

## Example 6

To 1 ℓ of a culture fluid obtained by cultivating human kidney cells and having the activity of 200 IU/mℓ was added 472 g of ammonium sulfate to make 70% saturation and to give a precipitate. The precipitate was dissolved in 15 mℓ of a 10mM phosphate buffer solution (pH 7.0) whose conductivity was adjusted to 50 mʊ/cm with NaCℓ. The solution was dialyzed in 3000 mℓ of the same 10mM phosphate buffer solution at 4°C for one night. After the dialysis was completed, the solution was centrifuged to give 14 mℓ of crudely purified urokinase solution having the activity of 10,700 IU/mℓ as a supernatant. The crudely purified urokinase solution having the activity of 150,000 IU was passed through a 1 mℓ column (0.9 cmφ) packed with Sepharose (Pharmacia Fine Chemicals Co.) having been chemically bonded (using known techniques employing cyanogen bromide) to 3.5 mg of 1G11a anti-urokinase monoclonal antibody per milliliter of Sepharose after the column was thoroughly washed with the same phosphate buffer solution as employed before.

After unadsorbed fraction was removed by washing the column with a phosphate buffer solution for rinsing, urokinase was eluted from the column with a 0.1M glycine-hydrochloric acid buffer solution (pH 2.8) whose conductivity was adjusted to 50 mʊ/cm with NaCℓ. The absorbancy of the eluted fraction was measured at 280 nm and the urokinase activity of the eluted fraction was measured in accordance with the standard fibrin plate method. The relationship between absorbancy or the urokinase activity and the elution volume is shown in FIG. 5. The purifying effects are shown in Table 1.

### Table 1

|  | Activity (IU/mℓ) | Protein Concen. (mg/mℓ) | Specific Activity (IU/mg) | Yield of Recovery (%) |
|---|---|---|---|---|
| Crudely Purified Urokinase Solution | 10,700 | 5.35 | 2,000 | - |
| Eluted Fraction | 43,500 | 0.332 | 131,000 | 93 |

As shown in Table 1, the specific activity increased about 65 times in one affinity chromatography step, and urokinase having a high purity was obtained with a high yield of recovery. The total yield of recovery in the chromatography with respect to activity was 98%.

### Example 7

To 25 ℓ of human urine (8 IU/mℓ) was added 5 g of polyacrylonitrile powder and the powder was suspended for 20 minutes. The suspension was centrifuged to collect the powder which adsorbed urokinase. The powder was suspended

in 40 ml of a 4% by weight aqueous ammonium solution to desorb urokinase, and the supernatant of the suspension was centrifuged to recover urokinase. The urokinase was dialyzed at 4°C for one night in a 10mM phosphate buffer solution (pH 7.0) whose conductivity was adjusted to 50 mʊ/cm with NaCl to give 40 ml of crudely purified urokinase solution (3,900 IU/ml). The crudely purified urokinase solution having the activity of 150,000 IU was passed through a 1 ml column (0.9 cm0) packed with polyacrylamide beads (Affi-Gel 701, Bio.Rad Co., U.S.A.) having been chemically bonded (using known techniques employing glutaraldehyde to activate the amino groups of the carrier prior to formation of a covalent bond) to 3.5 mg of 2C3i anti-urokinase monoclonal antibody per milliliter of the polyacrylamide after the column was thoroughly washed with the same phosphate buffer solution (pH 7.0) as employed in the dialysis.

After unadsorbed fraction was removed by washing ate the column with a phosphate buffer solution for rinsing, urokinase was eluted from the column with a 4% by weight $NH_4OH$ solution (pH 11) whose conductivity was adjusted to 50 mʊ/cm with NaCl. The purifying effects are shown in Table 2.

<div align="center">Table 2</div>

|  | Activity (IU/ml) | Protein Concen. (mg/ml) | Specific Activity (IU/mg) | Yield of Recovery (%) |
|---|---|---|---|---|
| Crudely Purified Urokinase Solution | 3,900 | 21.1 | 185 | - |
| Eluted Fraction | 38,500 | 0.36 | 107,000 | 91 |

The total yield of recovery in the chromatography with respect to activity was 95%.

## Example 8

980 mℓ of a culture fluid (189 IU/mℓ) obtained by cultivating human kidney cells was passed through a 1 mℓ column (0.9 cm∅) packed with the granular cellulose having been chemically bonded (using known techniques employing cyanogen bromide) to 3.5 mg of 5G4i anti-urokinase monoclonal antibody per milliliter of the cellulose after the column was thoroughly washed with the same 10mM phosphate buffer solution (pH 7.0) employed in Example 7.

After the unadsorbed fraction was removed by washing the column with a phosphate buffer solution for rinsing, urokinase was eluted from the column with a 6M guanidine hydrochloric acid buffer solution (pH 3.1) whose conductivity was adjusted to 50 m℧/cm with NaCℓ. The purifying effects are shown in Table 3.

### Table 3

| | Activity (IU/mℓ) | Protein Concen. (mg/mℓ) | Specific Activity (IU/mg) | Yield of Recovery (%) |
|---|---|---|---|---|
| Culture Liquid | 189 | 1.21 | 156 | – |
| Eluted Fraction | 20,000 | 0.195 | 102,600 | 97 |

The total yield of recovery in the chromatography concerned with the activity was 99%. As shown in Table 3, the specific activity increased about 650 times in one step.

## Example 9

The crudely purified urokinase solution (180,000 IU) obtained by the same method as in Example 6 was passed through a 1 mℓ column (0.9 cmℓ) packed with granular dextran which had been chemically bonded (using known techniques employing cyanogen bromide) to 3.5 mg of 6G8u anti-urokinase monoclonal antibody per milliliter of dextran after the column was washed with the same 10mM phosphate buffer solution (pH 7.0) as employed in Example 6.

After unadsorbed fraction was removed by washing the column with a phosphate buffer solution, urokinase was eluted from the column with an 8M aqueous urea solution (pH 7.0) whose conductivity was adjusted to 50 m℧/cm with NaCℓ. The purifying effects are shown in Table 4.

Table 4

|  | Activity (IU/mℓ) | Protein Concen. (mg/mℓ) | Specific Activity (IU/mg) | Yield of Recovery (%) |
|---|---|---|---|---|
| Crudely Purified Urokinase Solution | 13,100 | 5.7 | 2,300 | - |
| Eluted Fraction | 39,400 | 0.31 | 127,000 | 95 |

The total yield of recovery in the chromatography with respect to activity was 100%.

## Example 10

Nine mℓ of the purified urokinase solution (20,000 IU/mℓ) obtained in Example 8 was dialyzed at 4°C for one night in 2 ℓ of a 10mM phosphate buffer solution (pH 7.0) whose conductivity was adjusted to 50 mʊ/cm with NaCℓ. After the dialysis was completed, the solution was centrifuged to give 8.5 mℓ of a urokinase solution having the activity of 19,000 IU/mℓ. The urokinase solution (160,000 IU) thus obtained was passed through the same column as used in Example 9 after the column was thoroughly washed with the same 10mM phosphate buffer solution (pH 7.0) employed in Example 9.

After the unadsorbed fraction was removed by washing the column with a phosphate buffer solution, urokinase was eluted from the column with a 0.1M glycine-hydrochloric acid buffer solution (pH 2.8) whose conductivity was adjusted to 50 mʊ/cm with NaCℓ.

The absorbancy of the eluted fraction at 280 nm and the urokinase activity of the eluted fraction were measured, and the results are shown in FIG. 6. The purifying effects are shown in Table 5.

### Table 5

|  | Activity (IU/mℓ) | Protein Concen. (mg/mℓ) | Specific Activity (IU/mg) | Yield of Recovery (%) |
|---|---|---|---|---|
| Starting Urokinase Solution | 19,000 | 0.186 | 102,200 | − |
| Eluted Fraction | 75,000 | 0.524 | 143,000 | 94 |

The total yield of recovery in the chromatography with respect to the activity was 98%. As shown in Table 5, urokinase having a higher purity can be obtained by purifying a urokinase-containing solution with two kinds of columns, that is, the carriers packed therein were bonded with different anti-urokinase monoclonal antibodies, 5G4i and 6G8u.

<u>Comparative Example</u>

The affinity of 6G8u anti-urokinase monoclonal antibody for DEAE ion exchanger was measured. For the comparison, the affinity of IgG antibody which was obtained by purifying a normal mouse serum (conventional antisera) was measured by the same method. The results are shown in Table 6.

<u>Table 6</u>

| Conductivity (mʊ/cm) | Amount of Binding Antibody (%) | |
|---|---|---|
| | 6G8u Anti-urokinase Monoclonal Antibody | Conventional Antiserum |
| 0 | 100 | 13 |
| 5 | 100 | 5 |
| 10 | 100 | 2 |
| 15 | 95 | 0 |

The above description presents fusion of mouse B cells with cells of a mouse myeloma line. While mouse and rat B cells give a high percentage of stable fusions with most mouse myeloma lines, rabbit, human and frog B cells may also be used. Similarly, any of the several myeloma cell lines may be used, including P3x63Ag8, P3/NSI/1-Ag4-1, Sp2/0-Ag14 and S194/5.XX0.BU.1. It is to be understood that the present invention is intended to encompass all such fusions and others, which result in hybridomas capable of producing anti-urokinase monoclonal antibodies.

We claim:

1. A process for producing anti-urokinase antibodies, comprising: fusing an anti-urokinase antibody-producing cell and a myeloma cell to provide a fused cell hybrid, propagating said hybrid and collecting anti-urokinase antibodies produced by said hybrid, wherein said anti-urokinase antibody-producing cell is obtained from an animal immunized with human urokinase having a molecular weight of about 54,000 daltons.

2. The process of claim 1 wherein said monoclonal antibody belongs to the IgGl subclass and has a molecular weight of from 140,000 to 180,000 daltons and an isoelectric point of from 5.0 to 6.5.

3. The process of claim 1 wherein said hybrid is propagated in vitro.

4. The process of claim 1 wherein said hybrid is propagated in vivo.

5. The process of claim 1 wherein said anti-urokinase antibody-producing cell is selected from the group consisting of spleen cells and lymph node cells.

6. The process of claim 5 wherein said spleen is mouse spleen and said myeloma is mouse myeloma.

7. A process for producing anti-urokinase antibodies, comprising: injecting a BALB/c mouse with a human urokinase having a molecular weight of about 54,000 daltons to induce formation of anti-urokinase antibody-producing cells of said mouse, forming a fused cell hybrid of said anti-urokinase antibody-producing cells with P3x63Ag8Ul myeloma cells, culturing said hybrid in *vitro* in selective HAT medium to isolate said hybrid, propagating said isolated hybrid and harvesting the antibodies produced by said hybrid.

8. The process of claim 7 wherein said hybrid is propagated in *vitro*.

9. The process of claim 7 wherein said hybrid is propagated in *vivo*.

10. Anti-urokinase antibodies produced by the method of claim 1.

11. A method for diagnostically testing for urokinase, comprising: mixing anti-urokinase antibodies produced by the method of claim 1 with a solution suspected of containing urokinase, and detecting the interaction of said antibodies and urokinase.

12. A method for separating urokinase from a biological sample, comprising: contacting a biological sample containing urokinase with an antibody produced by the method of claim 1 and having affinity for said urokinase to form an antibody-urokinase complex, and separating the antibody-urokinase complex from the biological sample.

13. The method of claim 12, further comprising dissociating the antibody-urokinase complex to obtain purified urokinase.

14. A method for separating and recovering anti-urokinase monoclonal antibodies from a fluid, comprising: contacting a fluid containing anti-urokinase monoclonal antibodies produced by the method of claim 1 with an insoluble matrix having urokinase attached thereto to form a monoclonal antibody-urokinase complex, separating the antibody-urokinase complex from the fluid, and dissociating the antibody-urokinase  complex to obtain purified urokinase.

15.    A monoclonal antibody belongs to the IgGl subclass and has a molecular weight of from 140,000 to 180,000 daltons and an isoelectric point of from 5.0 to 6.5.

FIG.1

0084344

FIG.2

Passing Through    Washing   Elution   Re-Washing

Elution Volume (ml)

Absorbancy

Relative Affinity for Antigen

0084344

# FIG.3

# FIG.4

# FIG.5

Passing Through | Washing | Elution | Re-Washing

A280 / Absorbancy

(IU/ml) / Activity of Urokinase

Elution Volume (ml)

# FIG.6

Passing Through | Washing | Elution | Re-Washing

A280 / Absorbancy

(IU/ml) / Activity of Urokinase

Elution Volume (ml)